# EUROPÄISCHE PATENTSCHRIFT

(11) **EP 1 762 231 B1**
(45) Veröffentlichungstag und Bekanntmachung des Hinweises auf die Patenterteilung: **27.10.2010**
(21) Anmeldenummer: 05018093.4
(22) Anmeldetag: 19.08.2005
(51) Int. Cl.: A61K 9/32

(54) **Magnesium-Mikrotabletten mit verzögerter Freisetzung**
Sustained release magnesium microtablets
Micro-comprimes de magnésium à libération prolongée

(43) Veröffentlichungstag der Anmeldung: 14.03.2007
(73) Patentinhaber: Verla-Pharm Arzneimittelfabrik Apotheker H.J. von Ehrlich GmbH & Co. KG, D-82327 Tutzing (DE)
(72) Erfinder: Hopf, Günter, 82327 Tutzing (DE)
(74) Vertreter: Müller-Boré & Partner Patentanwälte

(56) Entgegenhaltungen:
- WO-A-01/24777
- DE-A1- 3 830 563
- DE-C1- 4 201 504
- US-A- 4 797 287
- US-A- 5 320 852
- US-A1- 2005 181 052
- US-B1- 6 355 676

## Beschreibung

Die vorliegende Erfindung betrifft Magnesium-Mikrolacktabletten mit verzögerter Freisetzung, d.h. Magnesium-Retard-Präparate, ein daraus gebildetes regelmäßiges Schüttgut sowie mit einem solchen Schüttgut befüllte Beutel sowie ein Verfahren zur Herstellung solcher Mikrotabletten.

Zur Therapie eines Magnesiummangels sind verschiedene Magnesiumverbindungen im Handel, deren Tagesdosis sich zwischen 100 und 400 mg Magnesium bewegt. Als handelsübliche Magnesiumpräparate stehen derzeit ausschließlich schnell freisetzende Arzneiformen, wie Brause-, Kau- und Lutschtabletten, Kapseln und Dragees zur Verfügung. Aufgrund der schnellen und vollständigen Wirkstofffreisetzung dieser Präparate sollte zur optimalen Aufnahme des eingenommenen Magnesiums die Dosis in drei kleinere Einnahmemengen über den Tag verteilt werden. Eine solche Einnahmeempfehlung steht jedoch meistens der Patientencompliance entgegen. So wird, entgegen der Einnahmeempfehlung, die Tagesdosis oft auf einmal genommen. Bedingt durch die schlechtere Resorption geht allerdings ein Teil der Dosis verloren. Durch diesen nicht resorbierten Teil der applizierten Dosis kommt es häufig zu Nebenwirkungen, welche sich vor allem in Durchfall äußern.

Um bei einmaliger täglicher Einnahme der Gesamttagesdosis eine vollständige Resorption zu erhalten, das Auftreten unerwünschter Nebenwirkungen zu unterbinden und gleichzeitig die Patientencompliance zu erhöhen, ist die Bereitstellung entsprechender Magnesium-Retard-Präparate wünschenswert. Solche Präparate sollten die Anforderung einer Einmaldosis mit einer Magnesiummenge von etwa 15 mmol entsprechend 365 mg Magnesium, eine verzögerte Freisetzung über 6 bis 8 Stunden sowie den Einsatz von Magnesiumsalzen mit optimalen Resorptionseigenschaften erfüllen.

Dabei erweist sich beispielsweise bei Magnesium-L-aspartat-hydrochlorid als Wirksubstanz mit hoher Bioverfügbarkeit der verhältnismäßig niedrige Magnesiumgehalt von 9,88 % Magnesium als äußerst nachteilig. Zur Erreichung der Zielvorgabe einer Einmaldosis bedeutet dies einen Wirkstoffeinsatz von bereits 3,69 g Magnesium-L-aspartat-Hydrochlorid. Auch ohne weiteren Einsatz von Hilfsstoffen würde sich somit als Einmaldosis eine Tablette ergeben, die aufgrund der Größe kaum mehr einnehmbar wäre und somit dem Ziel der vorstehend genannten optimalen Patientencompliance entgegenstehen würde.

Somit liegt der vorliegenden Erfindung die technische Aufgabe zugrunde, Magnesium-Retard-Präparate bzw. Mikrotabletten bereitzustellen, die sich durch ausgezeichnete Patientencompliance auszeichnen sollen, gleichzeitig aber die Anforderung einer Einmaldosis mit einer Magnesiummenge von etwa 12 bis 15 mmol entsprechend erfüllen.

Diese Aufgabe wird durch Bereitstellen der in den Ansprüchen gekennzeichneten Ausführungsformen gelöst.

Gelöst wurde das vorstehende Problem durch Herstellung von lackierten Mikrotabletten, die als Schüttgut bzw. regelmäßiges Granulat in einer Dosis patientenfreundlich eingenommen werden können.

Insbesondere wird eine Magnesium-Mikrolacktablette mit einem Tablettendurchmesser im Bereich von 1,6 bis 2,0 mm, vorzugsweise etwa 1,8 bis 1,9 mm, und einer Tablettenhöhe im Bereich von 2,75 bis 3,10 mm, vorzugsweise 2,90 bis 3,05 mm, bereitgestellt, welche einen mindestens ein Magnesiumsalz enthaltenden Kern und einen Lack bzw. Film bzw. Überzug darauf umfasst, wobei das mindestens eine Magnesiumsalz als pharmazeutisch wirksamer Bestandteil der Mikrolacktablette mindestens 70 Gew.%, vorzugsweise mindestens 75 Gew.-% des Kerns ausmacht und der Film durch Aufbringen einer vorzugsweise wässrigen Dispersion, welche einen oder mehrere Filmbildner enthält, auf den Kern erhältlich ist.

Der Magnesiumsalz enthaltende Kern der erfindungsgemäßen Mikrolacktablette weist vorzugsweise ein Einzelgewicht im Bereich von 8,00 bis 10,00 mg, mehr bevorzugt 8,25 bis 9,25 mg, auf, wobei das mindestens eine Magnesiumsalz als pharmazeutisch wirksamer Bestandteil mindestens 70 Gew.% des Kerns ausmacht. Des weiteren umfasst der Tablettenkern mindestens 15 Gew.-% eines Bindemittels, ausgewählt aus Hypromellose, Glycerinfettsäureester, wie z.B. Dynasan^{®}, oder allgemein üblichen Bindemitteln für die Direkttablettierung wie mikrokristalline Cellulose, z.B. Avicel^{®}, oder direkttablettierbarer Milchzucker, z.B. Tablettose^{®}. Den Rest des Tablettenkerns machen übliche Gleitmittel und Schmiermittel bzw. Formentrennmittel aus. Als Gleitmittel kann beispielsweise hochdisperses Siliciumdioxid (Aerosil 200) eingesetzt werden. Als Schmiermittel kann beispielsweise Magnesiumstearat verwendet werden. Der Tablettenkern ist das Ergebnis einer Direkttablettierung mit einem Magnesium-Wirkstoffanteil von mindestens 70 Gew.-%, vorzugsweise von mindestens 75 Gew.-%, mehr bevorzugt von mindestens 80 Gew.-%. Üblicherweise enthalten solche Mischungen bzw. Formulierungen gemäß dem Stand der Technik lediglich maximal 50 % Wirkstoff, um einen guten Verbund der Partikel und ausreichend Fließfähigkeit zu erreichen.

Das Magnesiumsalz als pharmazeutisch wirksamer Bestandteil im Tablettenkern der Mikrolacktablette unterliegt keiner wesentlichen Beschränkung. So können auch anorganische Magnesiumsalze wie Magnesiumoxid oder Magnesiumcarbonat eingesetzt werden. Bevorzugt sind jedoch organische Magnesiumsalze. Insbesondere kommen hierfür Magnesiumcitrat und Magnesiumaspartat in Frage. Besonders bevorzugt ist Magnesium-L-aspartat-Hydrochlorid.

In einer Ausführungsform der vorliegenden Erfindung ist der Tablettenkern aus 75-80 Gew.-% Magnesium-L-aspartat-Hydrochlorid, 15-25 Gew.-% Hypromellose und 0,5 bis 2 Gew.-% Additiven, ausgewählt aus Schmiermitteln und Gleitmitteln, aufgebaut.

Die Gesamtmasse der erfindungsgemäßen Mikrofilm- bzw. Lacktablette beträgt vorzugsweise etwa 8 mg bis 12 mg, mehr bevorzugt etwa 9 bis 10 mg, wobei die Gesamtmasse der Filmtablette vorzugsweise in Abhängigkeit vom Anteil des Magnesiumsalzes eingestellt wird. Der Anteil des Lacks bzw. Films liegt vorzugsweise in einem Bereich von etwa 1 Gew.-% bis etwa 15 Gew.-%, mehr bevorzugt etwa 2 Gew.-% bis etwa 10 Gew.-%, besonders bevorzugt etwa 4 Gew.-% bis etwa 9 Gew.-%, bezogen auf die Gesamtmasse der erfindungsgemäßen Lacktablette. Die Schichtdicke des Lacks bzw. Films beträgt üblicherweise 15 bis 50 µm, vorzugsweise 30 bis 40 µm.

Der Anteil des einen oder der mehreren Filmbildner liegt in einem Bereich von etwa 60 Gew.-% bis etwa 90 Gew.-%, bezogen auf die Gesamtmasse des Films. Der erfindungsgemäß verwendete Filmbildner unterliegt keiner besonderen Einschränkung, solange er pharmazeutisch verträglich ist und geeignet ist, die Wirkstoffdiffusion ausreichend lange zu retardieren, beispielsweise durch Quellen und Bilden von Mikroporen. Geeignete Beispiele sind Celluloseether wie Ethocel^{®}, Neutralpolymere auf (Meth)acrylat-Basis wie Eudragit^{®} NE 30D, ionische Polymere auf (Meth)acrylatBasis wie Eudragit^{®} RS bzw. RL oder Polyvinylacetat, stabilisiert mit PVP, wie Kollicoat^{®} SR. In einer bevorzugten Ausführungsform ist der Filmbildner ein Methacrylsäure-Acrylat-Copolymer bzw. ein Gemisch solcher Copolymere davon. Solche Filmbildner sind unter dem Handelsnamen Eudragit^{®} erhältlich. Insbesondere werden wasserunlösliche, quellbare Film- bzw. Lackbildner auf Basis von neutralen Methacrylsäureestern und einem geringen Anteil an Trimethylammoniumethylmethacrylatchlorid eingesetzt, erhältlich als Eudragit^{®} RL bzw. Eudragit^{®} RS. Für Eudragit^{®} RL beträgt das molare Verhältnis der quartären Ammoniumgruppen zu den neutralen Estergruppen 1 : 20 (entspricht etwa 50 mEq./100 g), für Eudragit^{®} RS 1 : 40 (entspricht etwa 25 mEq./100 g). Die Film- bzw. Lackmasse enthält darüber hinaus übliche Trennmittel wie z.B. Glycerinmonostearat und Weichmacher wie z.B. Triethylcitrat und gegebenenfalls Aromastoffe.

Ein weiterer Gegenstand der vorliegenden Erfindung betrifft ein Verfahren zur Herstellung der vorstehenden magnesiumhaltigen Mikrolacktabletten mit verzögerter Freisetzung, umfassend die Schritte:
(a) Mischen von mindestens einem Magnesiumsalz mit einem oder mehreren Bindemitteln und üblichen Hilfsstoffen,
(b) Tablettieren der erhaltenen Mischung unter Bildung von Tablettenkernen und
(c) Überziehen der Tablettenkerne mit einer Lackschicht, wobei dieser Lack durch Aufbringen einer vorzugsweise wässrigen Dispersion, welche eine oder mehrere Lackbildner enthält, auf den Kern erhalten wird.

Nach erfolgter Lackierung können die Mikrotabletten als regelmäßiges Granulat bzw. Schüttgut in Beutel bzw. Sachets bzw. Stickpacks abgefüllt werden, beispielsweise mit einem Füllgewicht im Bereich von jeweils 4,0 bis 6,0 g, vorzugsweise etwa 5,2 g. Ein solcher Beutel mit dem regelmäßigen Granulat aus den erfindungsgemäßen Mikrotabletten gewährleistet bei einer Einmaldosis eine Magnesiumaufnahme bzw. - dosis von etwa 10 bis 20 mmol in einer die Patientencompliance optimal erfüllenden Weise. Bezogen auf beispielsweise Magnesium-L-aspartat-Hydrochlorid gewährleistet ein solcher Beutel mit einem Füllgewicht im Bereich von jeweils 4,0 bis 6,0 g bei einer Einmaldosis eine Magnesiumaufnahme bzw. -dosis von etwa 11,5 bis 17,3 mmol. Die vorliegende Erfindung betrifft insofern auch die Verwendung der erfindungsgemäßen Mikrotabletten in Form eines regelmäßigen Schüttguts bzw. Granulats beispielsweise abgepackt in Beutel bzw. Sachets bzw. Stickpacks.

Das nachfolgende Beispiel wird angegeben, um die Erfindung näher zu erläutern, ohne diese dadurch zu beschränken.

### Beispiel

Die Herstellung untergliedert sich in folgende Herstellschritte:

### Einwaage der Mischung

Hierzu kam folgende Formulierung zur Anwendung:

| | **Substanz:** | **Menge Einzeldosis mg/Mikrotablette** |
|---|---|---|
| Wi= | Wirkstoff | |
| H= | Hilfsstoff | |
| Wi | Magnesium-L-aspartat-Hydrochlorid (9,88%) | 7,924 |
| H | Hypromellose | 1,981 |
| H | Schmiermittel und Gleitmittel | 0,096 |
| | | |
| | Gehalt bzw. Menge Magnesium/ Btl 15 mmol/4,67 g | |
| | | |
| | Summe: | 10 |

### Mischprozess

Der Mischvorgang erfolgt in einem herkömmlichen Mischer unter Zugabe der einzelnen Komponenten. Das resultierende Produkt ist freifließend und agglomeratfrei. Folgende IPC Werte wurden bestimmt:

| | |
|---|---|
| Schüttvolumen | 214-220 /100g |
| Relative Feuchte | 18 - 21 % |
| Absolute Feuchte | 4,25-4,34% |

### Tablettierung

Die Tablettierung erfolgt auf einer marktüblichen Tablettenpresse mit einer Geschwindigkeit von annähernd 50.000 Tabletten in der Stunde.

Folgende IPC Werte wurden hierbei erhalten:

| | |
|---|---|
| Durchmesser der Tabletten | 1,8 mm |
| Bruchfestigkeit der Tabletten | 12,4 - 18,6 N |
| Abrieb (Prüfmenge 20g/Zeitdauer 10 min) | nicht feststellbar |
| Einzelgewichte | 8,37-9,21 mg |
| Standardabweichung der Einzelgewichte | 1,6 - 2,4 |
| Tablettenhöhe | 2,83 - 2,95 mm |

Hervorzuheben sind die ausgezeichneten Tablettierungseigenschaften der Mischung und dies bei einem Wirkstoffanteil von annähernd 80%. Bei handelsüblichen Mischungen, die für eine Direkttablettierung empfohlen werden, liegt der Wirkstoffanteil üblicherweise bei 20%, bei optimalen Presseigenschaften des Wirkstoffs maximal bei 50%.

Dies ist besonders bemerkenswert, da aus dem Granulat sehr kleine Tabletten mit hoher Dosiergenauigkeit (s. Standardabweichung) gepresst werden können.

### Lackierung

Die verwendete Komponente Hypromellose bietet allein noch keinen ausreichenden retardierenden Effekt des Tablettenkerns, was unter anderem durch die sehr gute Wasserlöslichkeit des Magnesiumsalzes Magnesium-L-aspartat-Hydrochlorid und deren hoher Konzentration in der Tablettenrezeptur bedingt ist.

Zum Erhalt einer verzögerten Freisetzung wird deshalb eine Lackierung vorgesehen. Folgende maschinelle und technologische Probleme müssen dabei überwunden werden.
(i) Der kleine Kerndurchmesser der Tabletten wirft folgende Probleme auf:
   - Ungenügende Rolleigenschaften
   - Hohes Rollgewicht und damit erhebliche Abriebbelastung der Kerne
(ii) Die hohe Wasseraffinität der Kerne würde die Verwendung einer organischen Lackierlösung bedingen. Eine wässrige Lackierung ist aber gewünscht bzw. bevorzugt.

Es wurde erfindungsgemäß daher folgender automatisierter Prozess mit folgender Lackrezeptur, berechnet auf einen Ansatz von 180 kg Tablettenkerne, adaptiert:

| **Substanz:** | **Menge kg/Ansatz:** | **Menge TS in kg /Ansatz:** |
|---|---|---|
| Gemisch Eudragit RL 30 D/ Eudragit RS 30 D | 36,00 | 10,80 |
| Trennmittel | 1,08 | 1,08 |
| Weichmacher | 2,16 | 2,16 |
| VE-Wasser | 16,50 | |
| Aromen | 0,42 | |
| **Gesamtmenge:** | **56,16** | **14,04** |

Beachtenswert ist hierbei der Filmbildungsprozess, der auch nach der Lackierung beobachtbar ist. Dieser kann durch nachträgliches "Tempern" bei Temperaturen bis 40 Grad beschleunigt werden.

### Konfektionierung

Nach erfolgter Lackierung wird das Granulat in Beutel bzw. Stickpacks mit einem Füllgewicht von jeweils 5,2 g abgefüllt.

## Patentansprüche

1. Magnesiumhaltige Mikrolacktablette mit verzögerter Freisetzung, die einen Tablettendurchmesser im Bereich von 1,6 bis 2,0 mm und eine Tablettenhöhe im Bereich von 2,75 bis 3,10 mm aufweist, welche mindestens ein Magnesiumsalz enthaltenden Kern und eine darauf aufgebrachte Lackschicht umfasst, wobei das mindestens eine Magnesiumsalz als pharmazeutisch wirksamer Bestandteil der Mikrolacktablette mindestens 70 Gew.% des Gesamtgewichts der Tablette ausmacht und das Magnesiumsalz aus Magnesiumoxid, Magnesiumcarbonat, Magnesiumcitrat, Magnesiumaspartat und Magnesium-L-aspartat-Hydrochlorid ausgewählt ist.

2. Magnesiumhaltige Mikrotablette gemäß Anspruch 1, wobei der Magnesiumsalz enthaltende Kern der Mikrolacktablette ein Einzelgewicht im Bereich von 8,00 bis 10,00 mg aufweist.

3. Magnesiumhaltige Mikrotablette gemäß Anspruch 1 oder 2, wobei der Tablettenkern weiter mindestens 15 Gew.-% eines Bindemittels, ausgewählt aus Hypromellose, Glycerinfettsäureester, mikrokristalliner Cellulose oder direkttablettierbarem Milchzucker, insbesondere Hypromellose, enthält.

4. Magnesiumhaltige Mikrotablette gemäß einem der Ansprüche 1 bis 3, wobei der Tablettenkem aus 75-80 Gew.-% Magnesium-L-aspartat-Hydrochlorid, 15-25 Gew.-% Hypromellose und 0,5 bis 2 Gew.-% Additiven, ausgewählt aus Schmiermitteln und Gleitmitteln, aufgebaut ist.

5. Magnesiumhaltige Mikrotablette gemäß einem der Ansprüche 1 bis 4, wobei die Schichtdicke des Lacks 15 bis 50 µm beträgt.

6. Magnesiumhaltige Mikrotablette gemäß einem der Ansprüche 1 bis 5, wobei der Anteil des einen oder der mehreren Lackbildner in einem Bereich von etwa 60 Gew.-% bis etwa 90 Gew.-%, bezogen auf die Gesamtmasse des Lackes, liegt.

7. Magnesiumhaltige Mikrotablette gemäß einem der Ansprüche 1 bis 6, wobei der Lackbildner ein Methacrylsäure-Acrylat-Copolymer bzw. ein Gemisch davon ist.

8. Granulat, gebildet aus den magnesiumhaltigen Mikrotabletten gemäß einem der Ansprüche 1 bis 7.

9. Beutel, befüllt mit dem Granulat gemäß Anspruch 8 mit einem Füllgewicht im Bereich von 4,0 bis 6,0 g, so daß bei Einmaldosis eine Applikation einer Magnesiummenge im Bereich von 10 bis 20 mmol erreicht wird.

10. Verfahren zur Herstellung der magnesiumhaltigen Mikrolacktabletten mit verzögerter Freisetzung gemäß einem der vorhergehenden Ansprüche, umfassend die Schritte:
(a) Mischen von mindestens einem Magnesiumsalz, ausgewählt aus Magnesiumoxid, Magnesiumcarbonat, Magnesiumcitrat, Magnesiumaspartat und Magnesium-L-aspartat-Hydrochlorid, mit einem oder mehreren Bindemitteln und üblichen Hilfsstoffen,
(b) Tablettieren der erhaltenen Mischung unter Bildung von Tablettenkernen und
(c) Überziehen der Tablettenkerne mit einer Lackschicht, wobei dieser Lack durch Aufbringen einer vorzugsweise wässrigen Dispersion, welche eine oder mehrere Lackbildner enthält, auf den Kern erhalten wird.

## Claims

1. A magnesium-containing coated microtablet with sustained release, which has a tablet diameter in the range from 1.6 to 2.0 mm and a tablet height in the range from 2.75 to 3.10 mm, which comprises a core comprising at least one magnesium salt, and a coating layer applied thereto, wherein the at least one magnesium salt as pharmaceutically active ingredient of the coated microtablet accounts for at least 70% by weight of the total weight of the tablet and the magnesium salt is selected from magnesium oxide, magnesium carbonate, magnesium citrate, magnesium aspartate or magnesium L-aspartate hydrochloride.

2. The magnesium-containing microtablet according to Claim 1, wherein the magnesium salt-containing core of the coated microtablet has an individual weight in the range from 8.00 to 10.00 mg.

3. The magnesium-containing microtablet according to Claim 1 or 2, wherein the tablet core further comprises at least 15% by weight of a binder selected from hypromellose, glycerol fatty acid ester, microcrystalline cellulose or directly tablettable lactose, in particular hypromellose.

4. The magnesium-containing microtablet according to any of Claims 1 to 3, wherein the tablet core is composed of 75-80% by weight magnesium L-aspartate hydrochloride, 15-25% by weight hypromellose and 0.5 to 2% by weight additives selected from lubricants and glidants.

5. The magnesium-containing microtablet according to any of Claims 1 to 4, wherein the layer thickness of the coating is from 15 to 50 µm.

6. The magnesium-containing microtablet according to any of Claims 1 to 5, wherein the content of the one or more coating agent(s) is in the range from about 60% by weight to about 90% by weight based on the total mass of the coating.

7. The magnesium-containing microtablet according to any of Claims 1 to 6, wherein the coating agent is a methacrylic acid/acrylate copolymer or a mixture thereof.

8. Granules formed from the magnesium-containing microtablets according to any of Claims 1 to 7.

9. A bag filled with the granules according to Claim 8 with a packed weight in the range from 4.0 to 6.0 g, so that administration of an amount of magnesium in the range from 10 to 20 mmol is achieved with a single dose.

10. A process for producing the magnesium-containing coated microtablets with sustained release according to any of the preceding claims, comprising the steps:
(a) mixing at least one magnesium salt selected from magnesium oxide, magnesium carbonate, magnesium citrate, magnesium aspartate or magnesium L-aspartate hydrochloride, with one or more binders and conventional excipients,
(b) tabletting the resulting mixture to form tablet cores and
(c) coating the tablet cores with a coating layer, this coating being obtained by applying a preferably aqueous dispersion which comprises one or more coating agents onto the core.

## Revendications

1. Micro-comprimé contenant du magnésium à libération retardée, qui présente un diamètre de comprimé dans la plage de 1,6 à 2,0 mm et une hauteur de comprimé dans la plage de 2,75 à 3,10 mm, qui comprend au moins un noyau contenant un sel de magnésium et une couche de laque appliquée par-dessus, sachant qu'au moins un sel de magnésium constitue, en tant que constituant actif du point de vue pharmaceutique, au moins 70 % en poids du poids total du comprimé et que le sel de magnésium est choisi parmi l'oxyde de magnésium, le carbonate de magnésium, le citrate de magnésium, l'aspartate de magnésium et l'hydrochlorure du L-aspartate de magnésium.

2. Micro-comprimé contenant du magnésium selon la revendication 1, le noyau contenant le sel de magnésium du micro-comprimé présentant un poids individuel dans la plage de 8,00 à 10,00 mg.

3. Micro-comprimé contenant du magnésium selon la revendication 1 ou 2, le noyau du comprimé contenant en outre au moins 15 % en poids d'un liant, sélectionné parmi l'hypromellose, un ester d'acide gras de la glycérine, de la cellulose microcristalline ou du lactose transformable directement en comprimé, en particulier de l'hypromellose.

4. Micro-comprimé contenant du magnésium selon l'une quelconque des revendications 1 à 3, le noyau du comprimé étant constitué de 75-80 % en poids d'hydrochlorure de L-aspartate de magnésium, de 15-25 % en poids d'hypromellose et de 0,5 à 2 % en poids d'additifs, sélectionnés parmi les graisses et les lubrifiants.

5. Micro-comprimé contenant du magnésium selon l'une quelconque des revendications 1 à 4, l'épaisseur de couche de la laque étant de 15 à 50 µm.

6. Micro-comprimé contenant du magnésium selon l'une quelconque des revendications 1 à 5, la proportion d'un ou de plusieurs agents formateurs de laque se situant dans une plage d'environ 60 % en poids à environ 90 % en poids, par rapport à la masse totale de la laque.

7. Micro-comprimé contenant du magnésium, selon l'une quelconque des revendications 1 à 6, l'agent formateur de laque étant un copolymère acrylate - acide méthacrylique ou un mélange de ces derniers.

8. Granulé, formé à partir des micro-comprimés contenant du magnésium selon l'une quelconque des revendications 1 à 7.

9. Sachet, rempli du granulé selon la revendication 8, ayant un poids de remplissage dans la plage de 4,0 à 6,0 g, de telle sorte que l'on obtienne, en cas de dose unique, une application d'une quantité de magnésium dans la plage de 10 à 20 mmoles.

10. Procédé en vue de la fabrication de micro-comprimés contenant du magnésium à libération retardée selon l'une quelconque des revendications précédentes, comprenant les étapes :
(a) de mélange d'au moins un sel de magnésium, sélectionné parmi l'oxyde de magnésium, le carbonate de magnésium, le citrate de magnésium, l'aspartate de magnésium et l'hydrochlorure du L-aspartate de magnésium, avec un ou plusieurs liants et adjuvants usuels,
(b) de production de comprimés à partir du mélange obtenu avec formation des noyaux de comprimés et
(c) de revêtement des noyaux de comprimés à l'aide d'une couche de laque, cette laque étant obtenue par application sur le noyau d'une dispersion préférablement aqueuse, qui contient un ou plusieurs agents formateurs de laque.
